Europäisches Patentamt

(19) European Patent Office

Office européen des brevets

(11) Publication number: **0 382 031**
**A2**

# EUROPEAN PATENT APPLICATION

(21) Application number: **90101677.4**

(22) Date of filing: **28.01.90**

(51) Int. Cl.⁵: **A61K 39/395, A61K 37/04,
A61K 39/40**

(30) Priority: **10.02.89 US 309450**

(43) Date of publication of application:
**16.08.90 Bulletin 90/33**

(84) Designated Contracting States:
**AT BE CH DE DK ES FR GB GR IT LI LU NL SE**

(71) Applicant: **MILES INC.**
**Fourth and Parker Streets P.O. Box 1986**
**Berkeley California 94701(US)**

(72) Inventor: **Rousell, Ralph H.**
**5585 Coldwater Drive**
**Castro Valley, California 94552(US)**
Inventor: **Collins, Michael S.**
**2816 Wright Avenue**
**Pinole, California 94564(US)**

(74) Representative: **Dänner, Klaus, Dr. et al**
**c/o Bayer AG Konzernverwaltung RP Patente
Konzern**
**D-5090 Leverkusen 1 Bayerwerk(DE)**

(54) Monoclonal antibodies in immune serum globulin.

(57) Therapeutic efficacy of monoclonal antibodies can be enhanced by using plasma derived, polyclonal immune serum globulin as a vehicle for the monoclonal antibodies. Enhanced protective activity is demonstrated by using a combination of monoclonal antibodies specific to Pseudomonas aeruginosa in a plasma derived immune serum globulin (ISG), preferably an ISG having a high titer of plasma derived polyclonal antibodies to P. aeruginosa.

EP 0 382 031 A2

## MONOCLONAL ANTIBODIES IN IMMUNE SERUM GLOBULIN

### Background of the Invention

Field:

This disclosure is concerned generally with therapeutic antibody preparations and specifically with antibody preparations that are designed to have an enhanced protective effect when administered to a mammal.

Prior Art:

Immune serum globulin (ISG) preparations are routinely made from human plasma using known plasma fractionation techniques. For many years such ISG preparations have been administered to humans for both prophylactic and therapeutic effects. The early ISG preparations were administered intramuscularly and can be referred to as IMISG preparations. More recently, intravenously acceptable ISG preparations have been made available. These preparations can be referred to as IGIV preparations. See, for example, U.S. Patents 4,396,608 and 4,499,073 or 4,309,262.

ISG preparations include antibodies specific to many different antigens. In preparing an ISG for prophylactic or therapeutic uses, it is highly desirable to have a wide variety of antibodies to increase the likelihood of having at least some of the antibodies that will be effective or protective against a given antigenic pathogen. Such variety is commonly assured by preparing ISG from pools of plasma obtained from at least 1,000 blood plasma donors.

In some cases, where a known infection is to be prevented or treated, one can screen plasma donations for plasma having a higher than average titer for a given antibody and then forming a pool of plasma from those selected plasma donations. When such pools are fractionated using conventional techniques, they will yield an ISG having not only a wide variety of antibodies but also a higher than normal titer of antibodies of the type selected. These ISG preparations are referred to as hyperimmune products and several are commercially available (e.g. hyperhepatitis, hypertetanus, hyper CMV, hyper VZ, hyper RSV, hyper Pseudomonas, etc.). These hyperimmunes can be and are given prophylactically and therapeutically for a given infection (hepatitis, tetanus, etc.). The preparation of such hyperimmunes is described, for example, in U.S. 4,587,121 (Pseudomonas), U.S. 4,617,379 (CMV), U.S. 4,717,564 (VZ), and U.S. 4,717,766 (RSV).

Since the publication of the article by Köhler and Milstein, "Continuous Cultures of Fused Cells Secreting Antibody of Predefined Specificity," Nature 256:495-497 (1975), the production of monoclonal antibodies has become known and attention has been given to tailor-making high titer antibody preparations directed to a very specific antigen or a few antigens of a specific pathogen. This is being done with the hope that a highly effective prophylactic or therapeutic product will be possible for use against a very specific infection.

To avoid confusion regarding terminology, antibodies made from single cells such as the hybridomas of Köhler et al. or the later transformed cells of others (e.g. U.S. Pat. No. 4,446,465 to M. Lostrom) are all referred to as monoclonal antibodies, even if several different such antibodies are combined in a so-called cocktail (e.g. as in U.S.S.N. 734,624 filed May 15, 1985, filed in the name of A. Siadak et al.) describing a collection of monoclonal antibodies that bind to different antigenic determinants of Pseudomonas aeruginosa. Plasma derived antibodies are referred to as polyclonal, to describe both their wide variety and their plasma source. This also distinguishes them from monoclonal antibodies (MoAbs).

By their very nature, each monoclonal antibody is directed against and binds with a single epitope on a micro-organism or other antigen. Depending upon the nature and site of the epitope, the antibody may be defined as protective or not protective.

Monoclonal antibodies (MoAbs) directed against a single epitope on the surface of the bacterium or a virus can bring about the destruction of the micro-organism concerned. However, the MoAb is directed against a single epitope, and thus any organism(s) lacking the epitope will survive. Thus, a process of natural selection can bring about the emergence of resistant strains.

EP 0 382 031 A2

Summary of Invention

It has been found that the above problem can be avoided by insuring that a protective MoAb is not given alone, but in combination with a polyclonal immune serum globulin preparation. Although an artificial mixture of antibodies could be prepared using a rather wide variety of MoAbs, this method is impractical, being time consuming, expensive, and hopelessly inefficient when compared with the human polyclonal humoral immune system. Therefore it was found that the best method of minimizing the risk of emergence of resistant strains of organisms is to administer the monoclonal antibodies or MoAb cocktails as additives to an immune globulin preparation preferably an ISG intended for intravenous administration. Thus, the present disclosure is concerned with therapeutically effective pharmaceutical preparations comprising monoclonal antibodies and an immune serum globulin. The preparation may be in lyophilized form ready of aqueous reconstitution or already in aqueous form at a pH ranging from about 3.5 to 8.0, preferably about 3.5 to 5.0 as in U.S. 4,396,608.

Examples of the compatibility and activity of the MoAbs in combination with intravenous immune globulin, are shown below. The monoclonal antibodies used were prepared by Cutter Biological, Miles Inc. and the intravenous immune globulin was manufactured by Cutter Biological from pools of plasma which had high titers directed against Pseudomonas organisms. The plasma-derived preparation is known as Gamimune® Pseudomonas IGIV, pH 4.25. See for example U.S. Pat. 4,587,121 or U.S. Pat. 4,801,450 both to M.S. Collins and R.E. Roby.

3

## Example 1

Combination of Pseudomonas IGIV, pH 4.25 lot PR 3011 and IgM MoAb 2SC lot 3556-61-7 prophylaxis of Pseudomonas aeruginosa immunotype 1, murine burn wound sepsis.

Immunoglobulin 3 h pre-infection

| 2SC, mg/kg | PS-IGIV, mg/kg | No. dead/total[a] | % |
|---|---|---|---|
| 0 | 0 | 10/10 | 100 |
| 0 | 3.3 | 7/10 | 70 |
| 0 | 10.0 | 1/10 | 10 |
| 0 | 30.0 | 1/10 | 10 |
| 0.004 | 0 | 7/10 | 70 |
| 0.040 | 0 | 7/10 | 70 |
| 0.400 | 0 | 1/10 | 10 |
| 0.004 + | 3.3 | 2/10 | 20 |
| 0.040 + | 10.0 | 2/10 | 20 |
| 0.400 + | 30.0 | 1/10 | 10 |

[a] Challenged in burn site with $1.48 \times 10^5$ cfu P. aeruginosa 1369

Comment: Mortality in mice treated either with 3.3 mg PS-IGIV/kg or 0.004 mg 2SC/kg was 70%; when 3.3 mg PS-IGIV was combined with 0.004 mg 2SC/kg, mortality fell to 20%. With 2SC treatment alone (all 3 dosage levels) 15 of 30 (50%) mice died. With PS-IGIV treatment alone (all 3 dosage levels) 9 of 30 (30%) mice died. With the combination of 2SC and PS-IGIV (all 3 dosage levels) 5 of 30 (16.7%) mice died.

| Example 2 | | | |
|-----------|--|--|--|
| Combination of PS-IGIV 4.2 lot PR 3011 and IgM MoAb 1C1 lot 3722-26 prophylaxis of Pseudomonas aeruginosa-staphylococcus aureus polymicrobic murine burn wound sepsis. | | | |
| Immunoglobulin 3 h pre-infection | | | |
| 1C1, mg/kg | PS-IGIV, mg/kg | No. dead/total[a] | % |
| 0 | 0 | 8/10 | 80 |
| 0 | 3.3 | 5/10 | 50 |
| 0 | 10.0 | 1/10 | 10 |
| 0 | 30.0 | 3/10 | 30 |
| 0.004 | 0 | 5/10 | 50 |
| 0.040 | 0 | 0/10 | 0 |
| 0.400 | 0 | 1/10 | 10 |
| 0.004 + | 3.3 | 2/10 | 20 |
| 0.040 + | 10.0 | 1/10 | 10 |
| 0.400 + | 30.0 | 0/10 | 0 |

[a] Challenged in burn site with 113 cfu of P. aeruginosa immunotype 3 ATCC27314 and 160 cfu of S. aureus ATCC 14154.
Comment: Overall mortality in mice treated with MoAb 1C1 was 9 of 30 (30%) and 6 of 30 (20%) in mice treated with PS-IGIV alone. Mortality fell to 3 of 30 (10%) in mice given both immunoglobulins.

| Example 3 | | | |
|---|---|---|---|
| In vivo neutralization of exotoxin A in mice treated with a combination of IgG MoAb 8B9 (ATCC Accession No. CRL8833) and PS-IGIV. | | | |
| Immunoglobulin 3 h pre-infection | | | |
| 8B9, mg/kg | PS-IGIV, mg/kg | No. dead/total[a] | % |
| 0 | 0 | 7/10 | 70 |
| 0 | 33 | 10/10 | 100 |
| 0 | 100 | 10/10 | 100 |
| 0 | 300 | 0/10 | 0 |
| 0.004 | 0 | 10/10 | 100 |
| 0.040 | 0 | 4/10 | 40 |
| 0.400 | 0 | 2/10 | 20 |
| 0.004 + | 33 | 10/10 | 100 |
| 0.040 + | 100 | 0/10 | 0 |
| 0.400 + | 300 | 1/10 | 10 |

[a] Challenged by the intraperitoneal route with 0.3 ug exotoxin A (List Labs).

Comment: Overall mortality in mice treated with 8B9 alone was 16 of 30 (53.3%) and 20 of 30 (66.7%) in mice treated with PS-IGIV alone. Mortality fell to 11 of 30 (36.7%) in mice treated with both immunoglobuli s.

| Summary of in vivo studies with 2SC, 1C1, 8B9 and PS-IGIV | | |
|---|---|---|
| Treatment | No. dead/total | % |
| Control | 25/30 | 83.3% |
| MoAb only | 37/90 | 41.1% |
| PS-IGIV 4.2 only | 38/90 | 42.2% |
| MoAb + PS-IGIV | 19/90 | 21.1% |
| Comment: Overall, treatment with either MoAb or PS-IGIV reduced mortality approximately 2-fold. Combined therapy with both MoAb and PS-IGIV reduced overall mortality approximately 4-fold. This result suggests a least an additive protective activity with the combination of MoAb's and the IGIV. | | |

## Example 4

### Opsonic activity of IgM MoAb 2SC and PS-IGIV, pH 4.25 against P. aeruginosa immunotype 1-1369

| 2SC | $Log_{10}$ cfu reduction at 100 min[a] PS-IGIV ug IgG/mL | | |
|---|---|---|---|
| Ng IgM/mL | 0 | 50 | 200 |
| 0 | 0.00 | 0.20 | 1.06 |
| 50 | 0.30 | 0.46 | 1.20 |
| 200 | 1.35 | 1.85 | 1.55 |

[a] The assay employe 2% guinea pig serum as a complement source. The cfu reduction represents the $log_{10}$ reduction seen with 2% guinea pig serum minus the cfu reduction obtained when heated 2% guinea pig serum (complement inactivated) was used.

Comment: The numbers within the dotted line box represent cfu seen when both immunoglobulin preparations were present in the assay. In each case, the immunoglobulin combination promoted more killing of P. aeruginosa than either immunoglobulin alone.

From the examples given above, it can be concluded that IGIV preparations could well serve as a vehicle for MoAb's, for in no case was a loss of functional activity of either preparation seen. There was in each case at least an additional interaction.

While these studies utilized hyperimmune Pseudomonas IGIV, pH 4.25, it is thought that even a non-hyperimmune intravenous immune globulin could equally well be utilized as a vehicle for either a single monoclonal antibody or a cocktail of monoclonal antibodies, especially in those situations in which absolute definition of the infecting organism has not been obtained prior to initiation of treatment being required. As used herein, it is intended that the expression monoclonal antibodies include antibodies produced from hybridomas, transformed cell lines or variations thereof. Such antibodies may be human, of a non-human type (e.g. murine) or chimeric in nature.

Such monoclonal antibodies may bind with surface serotypio antigens or soluble toxins secreted by a microorganism such as Pseudomonas aeruginosa exotoxin A, staphylococcal toxins, etc. Such cell surface antigens and soluble toxins are well known to those skilled in the art and the present disclosure contemplates virally any combination of such antibodies with a polyclonal ISG that results in a protective effect when administered as a pharmaceutically acceptable preparation. As used herein, protective means capable of reducing cumulative mortality in infected animals.

The term pharmaceutically acceptable preparation refers to a preparation that can be administered either orally, topically or parenterally without adverse effect on an animal. Various excipients may be included and these include amino acids (e.g. glycine), carbohydrates (e.g. maltose, sucrose, etc.) and proteins (e.g. albumin, gelatin, etc.).

### Other Examples

The principles of this disclosure would cover a broad array of monoclonal antibody/ISG combinations. For example, monoclonal antibodies directed against viral antigens such as human cytomegalovirus (CMV) polypeptides (Rasmussen et al., J. of Virology, p. 274-280, Aug. 1985), may be combined with the ISG or IVIG. Also, monoclonals that bind with fungal antigens such as Cryptococcus neoformans capsular polysaccharide (Dromer et al., Infection and Immunity, p. 749-752, March, 1987), parasite antigens such as Pneumocystis carinii glycoprotein (Gigliotti et al., J. of Clin. Invest., Vol. 81, p. 1666-1668, 1988) and Naegleria fowleri (Lallinger et al., Infection and Immunity, pp. 1289-1293, May, 1987) may be similarly combined. Also monoclonals to bacterial antigens such as Proteus mirabilis flagella (Rutherford et al., U.S. Pat. 4,772,464), Escherichia coli J5 mutant lipopolysaccharide (Teng et al., Proc. Nat'l. Acad. Sci., Vol. 82, pp. 1790-1794, Mar. 1985), and Haemophilus influenzae type b capsular polysaccharide (Hunter et al., Lancet, p. 798 et seq., Oct. 9, 1982) could be similarly added to ISG or IGIV.

Given the above examples, it is thought that variations will occur to those skilled in the art. Accordingly, it is intended that the above examples should be construed as illustrative only and that the scope of the invention disclosed herein should be limited only by the following claims.

## Claims

1. A therapeutically effective pharmaceutical preparation comprising monoclonal antibodies and an immune serum globulin.

2. The preparation of Claim 1 including an excipient selected from an amino acid, a carbohydrate and a protein.

3. The preparation of Claim 2 where the protein is albumin or gelatin.

4. The preparation of Claims 1 - 3 wherein said monoclonal antibodies bind with at least one serotypic determinant of Pseudomonas aeruginosa or Pseudomonas aeruginosa exotoxin A and an immune serum globulin.

5. The preparation of Claim 4 wherein the immune serum globulin is a hyperimmune Pseudomonas immune serum globulin.

6. The preparation of Claim 4 wherein the monoclonal antibody is selected from the group of antibodies that bind with Fisher serotypes 1, 2, 3, 4, 5, 6 and 7 of Pseudomonas aeruginosa and Pseudomonas aeruginosa exotoxin A.

7. The use of a preparation according to Claims 1 - 6 for the manufacture of a pharmaceutical.

8. A process for the manufacture of a pharmaceutical which comprises mixing monoclonal antibodies and an immune serum globulin.